# EUROPÄISCHE PATENTSCHRIFT

(11) **EP 3 035 892 B1**
(45) Veröffentlichungstag und Bekanntmachung des Hinweises auf die Patenterteilung: **03.08.2016**
(21) Anmeldenummer: 08856958.7
(22) Anmeldetag: 06.11.2008
(51) Int. Cl.: A61F 2/44, A61B 17/70

(54) **MODULARE PROTHESEN**
MODULAR PROSTHESES
PROTHÈSES MODULAIRES

(30) Priorität: 04.12.2007 DE 102007058301
(43) Veröffentlichungstag der Anmeldung: 29.06.2016
(73) Patentinhaber: Global Medical Consulting Gmbh, 94327 Bogen (DE)
(72) Erfinder: BERTAGNOLI, Rudolf, 94315 Straubing (DE)
(86) Internationale Anmeldenummer: PCT/DE2008/001831
(87) Internationale Veröffentlichungsnummer: WO 2009/071045

(56) Entgegenhaltungen:
- WO-A-2007/003438
- US-A- 5 674 296
- US-A1- 2003 204 261
- US-A1- 2005 049 707
- US-A1- 2007 073 311

## Beschreibung

Modulare Bandscheibenprothese bestehend aus mindestens einer an einem Wirbelkörper implantierbarer Appositionsplatte und einem mit der bzw. den Appositionsplatte/n lösbar befestigbaren Zwischenmodul, wobei die Appositionsplatte/n mit Mitteln wie Stiften, Krampen, Zahnungen, ausgebildet ist bzw. sind derart, daß sie auf Dauer, d.h. Revisionen überstehend, am Wirbelkörper fixierbar ist bzw. sind.

Modulare Prothesen sind bekannt. In der DE 695 16 603 T2, WO 2005/027800 A2 und WO 2005/094732 A1 sind beispielsweise Bandscheibenprothesen modularer Bauart beschrieben. Gemäß der WO 2005/094732 A1 wird die modulare Prothese in Form eines Baukastens aus einer Anzahl von Zwischenstücken und einer Anzahl daran lösbar befestigbaren Wirbelendplatten bzw. Wirbelendplatten unterschiedlicher Bauart für den betreffenden Patienten präoperativ zusammengestellt und dann implantiert. Notfalls wird auch interoperativ ein Austausch von Modulen stattfinden, wenn die anatomischen Gegebenheiten des Patienten es erfordern. Hierbei handelt es sich um die geeignete Zusammenstellung der Prothesenmodule vor oder auch während des chirurgischen Eingriffes.

Es bestehen jedoch oftmals Fälle, in denen der Eingriff wegen Abnutzung oder Zerstörung des Implantats oder anderer Gründe ggf. nach Jahren wiederholt werden muß. Die Implantation von Prothesen ist ein komplizierter Eingriff.

Um hier Abhilfe zu schaffen sind aus den US 2003/ 0204261 und US 2007/0073311 modulare Bandscheibenprothesen bekannt geworden, die derart gestaltet sind, daß die Appositionsplatten bei einer Revision implantiert bleiben. Damit bleibt zur Entlastung des Knochenmaterials der Wirbelkörper die Platten am Knochen fixiert und dient gleichzeitig als Halterung für die übrigen austauschbaren Module der Prothese, die die eigentlichen Funktionsmodule sind.

Der Erfindung liegt die Aufgabe zugrunde, dieses Konzept weiter zu verbessern.

Die Aufgabe wird erfindungsgemäß durch die Merkmale des Anspruchs 1 gelöst.

Die künstlichen Wirbelendplatten sind erfindungsgemäß mit Bohrungen, vorzugsweise Schrägbohrungen versehen, durch die aushärtbares Material mit osteoconductiven oder osteoinductiven Eigenschaften oder andere Kunststoffe in den Hohlräumen gespritzt werden kann, die zwischen der Appositionsplatte und dem Wirbelkörper verbleiben.

Aus der US 2005/0049707 ist zwar die Verwendung von Zement im Zusammenhang mit Bandscheibenprothesen bekannt, jedoch handelt es sich hierbei, um die Zementierung des Implantates an die Wirbelkörper. Derartige Befestigungen von Implantaten haben jedoch den Nachteil, dass aufgrund von Bewegungen und Erschütterungen Zementteile abbröckeln können, und, wie bei zementimplantierten künstlichen Hüftgelenken bereits bekannt, Lockerungen des Implantats die Folge sein können.

Im Gegensatz hierzu erfolgt die Fixierung der erfindungsgemäßen Wirbelendplatte mittels Stiften, Zahnungen, oder dergleichen, was eine dauerhafte Befestigung mit der Wirbelendplatte sicherstellt. Darüber hinaus werden gemäß der Erfindung nur die Unebenheiten der Wirbelkörperendflächen hinter der künstlichen Wirbelendplatte ausgeglichen, so dass die Krafteinleitung der künstlichen Wirbelendplatte auf die natürliche Wirbelendplatte über die gesamte Kontaktfläche gleichmäßig ist. Dieses bewirkt im Gegensatz zur bekannten Ausführung eine gleichmäßige Druckverteilung, die in Verbindung mit der mechanischen Fixierung zur besseren Stabilisierung der Wirbelendplatten und damit der gesamten Prothese führt.

Die austauschbaren Prothesenmodule, die eine Funktion im Körper ersetzen, werden mit einem fest verankerten Halterungsmodul verbunden, was mit gängigen Mitteln, wie Schrauben, Krampen, Bajonettverschlüssen, lösbare Verklebung, etc. in einer wieder lösbaren Art geschieht. Das hat den großen Vorteil, dass bei einer erforderlichen Erneuerung oder einem Austausch des Funktionsmoduls, dieser lediglich vom Halterungsmodul zu lösen und das neue austauschbaren Modul entsprechend anzubringen ist. Damit bleibt die Knochenimplantatzone beim Revisionseingriff unberührt und es kommt nicht zu einer operationsbedingten Reduktion der Knochensubstanz. Außerdem lässt sich eine zweite oder dritte Operation wesentlich schneller durchführen als im konventionellen Verfahren.

Die Erfindung hat den weiteren Vorteil, daß das auf Dauer zu verankernde Halterungsmodul zusätzlich für die Befestigung von Zusatzkomponenten, wie Zuggurtungsbänder, Dämpfer, etc., verwendet werden kann, wodurch eine weitere Entlastung des Knochenmaterials möglich ist.

Besonders vorteilhaft ist es, wenn das fest zu verankernde Halterungsmodul sowie die austauschbaren Modulkomponenten genormt sind. Auf die Weise kann der Chirurg ohne die Ausgestaltung der implantierten Prothese und insbesondere des als Halterung dienenden Moduls untersuchen zu müssen, von vornherein das passende austauschbare Modul bereitstellen und den Eingriff vornehmen. Damit wird vermieden, dass der Chirurg erst beim Eingriff bemerkt, dass die bereitgestellten Austauschmodule nicht mit dem im Patienten eingebrachten festen Halterungsmodul kompatibel sind, was für den Patienten eine Doppelbelastung bedeuten würde, da der Eingriff ergebnislos sein würde und wiederholt werden müsse.

Die Normierung hat außerdem den Vorteil, dass genormte Bausätze von modularen Prothesen und Zusatzkomponenten sowie die passenden Instrumente für die Implantation sich wirtschaftlich herstellen lassen und die Operation kann zu einem Routineeingriff reduziert werden.

Es werden jeweils für die verschiedenen Prothesen, wie Wirbel-, Bandscheiben-, Gelenk- und andere Prothesen sowie Zusatzkomponenten, wie Dämpfer, Zuggurtungsbänder, entsprechende Normen bestimmt.

Im Falle von Wirbelsäulen-Implantate können künstliche, Hülsen für Dorn- und Querfortsätze, Platten oder Halbhülsen für den Wirbelbogen oder um den Wirbelkörper sowie Muttern oder ähnliche Blöcke als Halterungsmodule verwendet werden. Die künstlichen Wirbelendplatten dienen zur lösbaren Verankerung von Wirbelkörper- oder Bandscheibenimplantaten, aber auch gleichzeitig zur Halterung von Zusatzkomponenten, wie Dämpfer, Bewegungsbegrenzungskomponenten, etc. Die künstliche Wirbelendplatte ersetzt das Knochenmaterial des Wirbelkörpers, indem sie als Verankerung für austauschbare Funktionsmodule dient. Bei einem späteren Revisionseingriff, werden die alten künstlichen Wirbelendplatten, die mit den natürlichen Endplatten der Wirbelkörper verbunden bleiben, nicht ausgetauscht, sondern sie bilden vielmehr eine mechanisch definierte Komponente, die eine genaue Platzierung und Fixierung von austauschbaren Modulen erlaubt, so daß bei einer Revision der chirurgische Eingriff sich im wesentlichen auf einen mechanischen Austausch des Funktionsmoduls reduziert. Damit wird die Knochenverankerung nicht gestört, das Knochenmaterial geschont und der Eingriff erheblich verkürzt.

Dadurch, dass bei einer Revision nicht die gesamte Prothese, z.B. Bandscheiben-, Wirbelkörper- interspinöses Implantat, sondern nur das Funktions- und ggf. Zusatzmodul ausgetauscht werden muß, ergeben sich wesentliche Vorteile, nämlich, daß bei einer Revision der Prothese für das Rückgrad dem Chirurgen weitgehende Freiheiten bestehen. Der erneute Eingriff ist nicht mehr zwingend vom Bauch- bzw. Vorderbereich des Patienten her durchzuführen, der aufgrund der vorgelagerten Organe Komplikationen bereitet. Das austauschbare Modul kann nämlich auch durch einen Zugang seitlich vorne oder vom Rücken des Patienten aus durchgeführt werden, was eine schonendere und komplikationsärmere Operation am menschlichen Körper bedeutet.

Das austauschbare Modul wird je nach Anwendung einteilig, mehrteilig, gelenkig, als Kompressionsfeder, als Puffer oder dergleichen ausgebildet sein. In jedem Fall werden die austauschbaren Module und Komponenten mit Mitteln zur lösbaren Verankerung mit einer oder mehreren künstlichen Haltemodulen ausgestattet. Als Mittel zur lösbaren Befestigung von Implantatmodulen werden Schraubverbindungen, Schwalbenschwanz- oder Bajonettverschlüsse, etc. vorgesehen. Lösbare Verklebung ist auch denkbar.

Ebenso erlaubt der Erfindungsgegenstand durch seine Modularität, Bewegungsmodule unterschiedlicher Materialpaarungen, Dämpfungseigenschaften oder Bewegungsfreiheitsgrade aufzunehmen.

Eine einfache Ausgestaltung der künstlichen Wirbelendplatten besteht aus zwei Endplatten, die mit bekannten Stiften, Zahnungen, etc. ausgestattet jeweils an einen Wirbelkörper auf Dauer fixiert werden. Eine derartige Ausführung hat eine geringe Höhe und bedarf somit beim Einsetzen einen kleineren Wirbelzwischenspalt und demzufolge eine geringere Streckung der Wirbelköper als bei herkömmlichen Gesamtimplantaten. Die Endplatten sind von vornherein mit normierten Mitteln ausgestattet, die zur lösbaren Befestigung von austauschbaren Modulen und ggf. zur Befestigung anderer Komponenten, wie Zuggurtungsbänder, Dämpfer, Bewegungs-Begrenzungsmittel, etc. dienen.

Das austauschbare Modul wird optimal an die zu ersetzende Funktion im Körper des Patienten ausgelegt und ebenfalls mit Mitteln zur Verankerung mit den Wirbelendplatten versehen, wie Schwalbenschwanzgegenstück, Bohrungen für Schrauben, etc.

Eine weitere Ausgestaltung der Erfindung sieht die Verbindung des austauschbaren Moduls mit einem Zuggurtungsband vor, das ebenfalls mit den permanenten Endplatten verbunden werden kann und als Teil der Bandscheibenprothese zur elastischen oder fixen Bewegungsbegrenzung dient. Bei Begrenzungen von Beugungs- und Rotationsbeweglichkeiten der Wirbelsäule können symmetrische oder asymmetrische Begrenzungsmittel bzw. -komponenten eingesetzt und bei Notwendigkeit leicht verändert werden, indem z.B. die Bewegungs-Begrenzungskomponenten einer Vorimplantation vom Halterungsmodul gelöst und gegen Komponenten anderer Eigenschaften Ausgetauscht werden.

Die künstlichen Wirbelendplatten sind erfindungsgemäss mit Bohrungen, vorzugsweise Schrägbohrungen versehen, durch die aushärtbares Material mit osteoconductiven oder osteoinductiven Eigenschaften oder andere Kunststoffe in den Hohlräumen gespritzt werden kann, die zwischen der Platte und dem Wirbelkörper verbleiben. Damit werden die Unebenheiten der Wirbelkörperendflächen hinter der künstlichen Wirbelendplatte ausgeglichen, so dass die Krafteinleitung der künstlichen Wirbelendplatte auf die natürliche Wirbelendplatte über die gesamte Kontaktfläche gleichmäßig ist.

Für interspinöse Prothesen oder Implantate werden Hülsen als feste Plattform vorgesehen. Diese Hülsen, beidseitig oder nur einseitig offen, werden über Dornfortsätze sowie auch Querfortsätze gestülpt und ebenfalls auf Dauer am Dorn- bzw. Querfortsatz fixiert. Eine Normierung dieser Hülsen bietet die gleichen oben bereits beschriebenen Vorteile. Längsseitig offene Hülsen werden für Wirbelbögen oder um den Wirbelkörper verwendet.

Die Erfindung ist auf alle Implantate anwendbar, bei denen mindestens ein Modul fest mit Knochenmaterial verankert und mindestens ein weiteres Modul mit dem ersten lösbar verbunden werden kann.

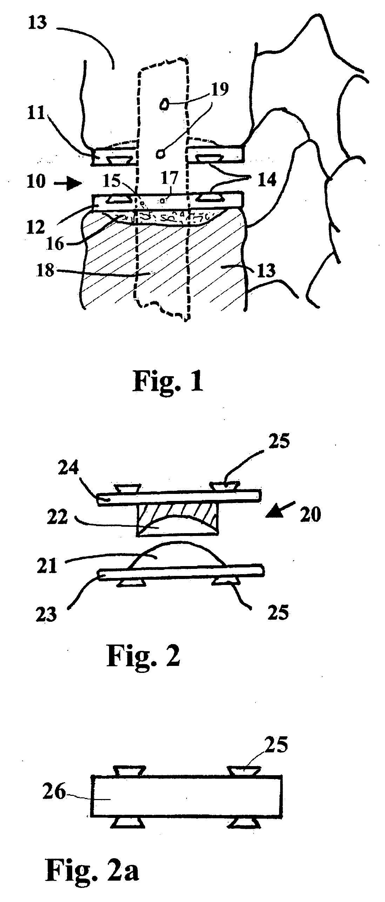

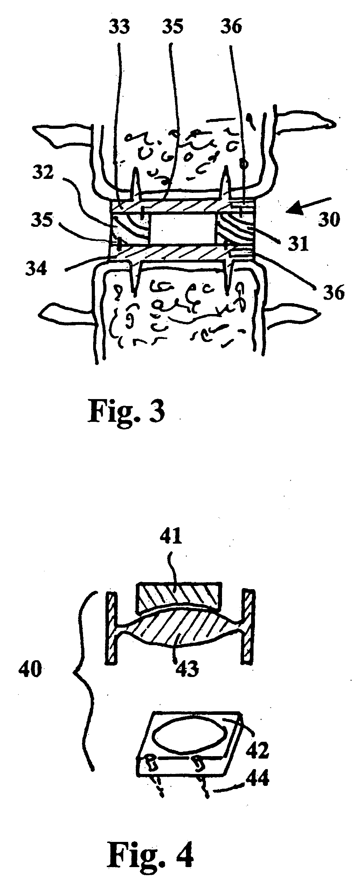

Die Erfindung wird anhand von in der Zeichnung schematisch dargestellten Ausführungsbeispielen näher erläutert. Es zeigen: Fig.1 bis 2a ein erstes Ausführungsbeispiel teils im Schnitt, Fig.3 bis 6 je ein weiteres Ausführungsbeispiel in Längs-, Teilschnitt. bzw. Draufsicht.

Die erfindungsgemäße Prothese besteht grundsätzlich aus einem Halterungsmodul, das in einem ersten Operationseingriff derart implantiert wird, dass es auf Dauer im menschlichen Körper verankert bleibt und so ausgestaltet ist, daß es weitere, die eigentliche Funktion der Prothese darstellende Module lösbar und austauschbar aufnimmt.

In Figuren 1-5 wird die Erfindung anhand von Bandscheibenprothesen beschrieben, die eine anschauliche Darstellung der Erfindung bieten. Natürlich ist das Prinzip für jede Prothese in ähnlicher Weise konstruierbar.

Nach der Entfernung der beschädigten Bandscheibe wird ein erstes, als Halterung 10 (Fig.1) dienendes Modul, bestehend aus zwei künstlichen Wirbelendplatten 11,12, eingesetzt und mittels Stiften, Krampen oder Verklebung jeweils mit den Wirbelkörpern 13 derart befestigt, dass sie auf Dauer mit den Wirbelkörpern 13 verbunden bleiben. Anschließend wird über in den Wirbelendplatten 11,12 vorgesehene Kanäle 15 aushärtbares, körperverträgliches Material mit osteoconductiven oder osteoinductiven Eigenschaften oder anderer Kunststoff unter die jeweilige künstliche Wirbelendplatte 11,12 eingespritzt, um den durch die annähernd konkave Wirbelkörperkontaktfläche gebildeten Holraum 16 auszufüllen, und damit eine gleichmäßige Druckverteilung zu erreichen. Damit ist die Vorbereitung zum Einsatz des zweiten bzw. austauschbaren Moduls, das die Funktion der Prothese übernimmt, beendet.

Ein in Längsschnitt dargestelltes austauschbares Modul 20 ist in Fig.2 gezeigt. Es besteht aus einem Halbkugelkopf 21 und einer mit dem Halbkugelkopf zusammenwirkenden Pfanne 22. Diese Gelenkkomponenten bilden jeweils mit einem Anschlußstück 23,24 eine Baueinheit. Das austauschbare Modul 20 wird nun zwischen die beiden künstlichen Wirbelendplatten 11,12 eingeschoben derart, dass die Schwalbenschwanzstege 25 des austauschbare Moduls 20 in die Schwalbenschwanznuten 14 der Wirbelendplatten 11,12 eingreifen und damit eine lösbare Verbindung zwischen Halterungsmodul 10 und austauschbarem Modul 20 bewirken.

Abschließend wird ein elastisches Band oder Zuggurtungsband 18 an in Schraubbohrungen 17 der künstlichen Wirbelendplatten 11,12 und ggf. zusätzlich an den Wirbelkörpern angeschraubt (19), um Zugkräfte auf die Bandscheibenprothese 10,20 auszuüben und gleichzeitig die Verankerung des austauschbare M0duls 20 zu sichern. Das Zuggurtungsband übernimmt dabei auch die Funktion einer Bewegungsbegrenzung in Beugungsrichtung.

Ist beispielsweise bei dem mit der implantierten Bandscheibenprothese 10,20 Patienten nach Jahren eine Veränderung erfolgt, derart, dass die Prothese erneuert werden muß, dann wird bei einem erneuten chirurgischen Eingriff nur das elastische Band 18 abgeschraubt und das austauschbare Modul 20 herausgezogen. Die künstlichen Wirbelendplatten 11,12 verbleiben in ihrer ursprünglichen Verankerung und werden ein neues austauschbares Modul aufnehmen.

Ist eine Revision wegen erforderlicher Versteifung der Wirbelkörper vorgesehen, dann wird ebenso verfahren, nur dass anstelle eines gelenkigen austauschbaren Moduls ein mit Schwalbenschwanzstegen 25 ausgerüsteter Distanzhalter 26, Fig.2a, als austauschbares Modul eingesetzt wird.

In Einzelfällen ist die Verwendung von nur einer künstlichen Wirbelendplatte und einem austauschbaren Modul möglich. Im Beispiel nach Figuren 1 und 2 würde die Bandscheibenprothese aus der Wirbelendplatte 12 z.B. in Verbindung mit dem austauschbaren Modulteil 21,23 bestehen, wobei das austauschbare Modulteil mit dem Halbkugelkopf 21 direkt in der Wölbung des gegenüberliegenden Wirbelkörpers 13 aufliegen wird.

Die Erfindung ist unabhängig von der Ausgestaltung der Module, insbesondere des austauschbaren Moduls. Die Wirbelendplatten sind mit zusätzlichen Mitteln, wie Schraubbohrungen 17 bzw. 36 (Fig.3) versehen, die die Verankerung von anderen notwendigen Komponenten erlauben, wie Stützelemente, Zuggurtungsbänder 18, Bewegungsbegrenzungs-Elementen, etc.

Es ist jedoch von großem Nutzen, wenn die Module, insbesondere die auf Dauer zu verankernden Halterungsmodule normiert sind. Damit steht einem gezielten Austausch von Funktionsmodulen nichts im Weg, indem der Chirurg die im Körper des Patienten verankerte Halterung von vornherein genau kennen kann. Dabei sind nicht notwendigerweise nur einheitliche Funktionsmodule zu verwenden. Diese können in ihrer Funktionsweise durchaus unterschiedlich sein, nur ihre Anbindung an die Halterungsmodule muß normiert und mit der Halterung kompatibel sein.

In Fig.3 ist ein Beispiel gezeigt, bei dem das austauschbare Modul 30 aus mindestens zwei nebeneinander anzuordnenden Modulkomponenten 31,32 besteht. Die austauschbaren Modulteile 31,32 bestehen jeweils aus einer Spirale oder ineinandergreifenden Doppelspiralen, deren Enden jeweils an die mit Schraubbohrungen versehenen Halterungsplatten 33,34 des ersten Moduls angeschraubt (35) werden. Die Spiralen ermöglichen Kippbewegungen. Diese Ausführung eignet sich für dorsalseitige Revisionsoperationen. Durch Lösen der Schrauben 35 lassen sich diese austauschbaren Modulteile 31,32 herausziehen und durch ein neues austauschbares Modul gleicher oder anderer Bauart, durch massive Blöcke im Falle der Versteifung oder elastischer Blöcke auswechseln. Eine Versteifung ist auch dadurch erreichbar, dass nach Entnahme des gelenkigen austauschbaren Moduls die beiden künstlichen Wirbelendplatten 33,34 direkt miteinander, z.B. durch Verkleben, Verschrauben, verbunden werden.

Bei der Ausführung gemäß Fig. 3 ist die Möglichkeit gegeben, dass die beiden austauschbaren Modulkomponenten 31,32 bei der Erstoperation, bei der die auf Dauer einzusetzenden Halterungsplatten 33,34 verankert werden, von vorne, z.B. vom Bauchraum aus implantiert werden. Während bei einer zweiten Operation die lösbaren Modulkomponenten 31,32 vom Rücken des Patienten aus ausgetauscht werden können.

Das Beispiel nach Fig.3 eignet sich auch zur Herstellung von Bandscheibenprothesen, die asymmetrische Beugewinkel ermöglichen, indem die austauschbaren Modulkomponenten 31 und 32 unterschiedliche Druckkräfte auf die künstlichen Wirbelendplatten 33,34 ausüben. Eine notwendige Veränderung der Beugungsmöglichkeiten ist auch ein Revisionsgrund, bei dem lediglich ein oder beide austauschbaren Modulkomponenten 31,32, durch neue anderer Federstärken ausgetauscht zu werden brauchen.

Bei anderen Gelenkprothesen, wie beispielweise das in Fig. 2 gezeigte Halbkugel-Pfannensystem 21,22, werden Beugungsbegrenzungen mit gesonderten, nicht dargestellte jedoch im Stand der Technik bekannte Mittel, wie elastische Puffer, Federn, oder formgebende Maßnahmen hervorgerufen. Diese können mit einer oder beiden Wirbelendplatten verbunden werden, oder aber auch integraler Bestandteil eines Zuggurtungsbandes sein. Es ist oft notwendig, die Beweglichkeitsgrenzen für den Patienten individuell und dabei auch asymmetrisch auszulegen. Es ist ebenfalls Bestandteil der Erfindung, diese Maßnahmen in das erfindungsgemäße Verfahren einzubeziehen. Die Mittel, um die Beuge-Extensionsbewegung und/oder die Seitenneigung und/oder die Rotation um die Hochachse symmetrisch oder asymmetrisch zu Begrenzen, werden wie das austauschbare Modul lösbar in das Prothesensystem integriert bzw. eingebaut. Muß im Laufe der Zeit, die Beweglichkeitsgrenze für eine oder mehrere Beugungsrichtungen verändert werden, so werden beim Revisionseingriff die betreffenden Mittel aus der Prothese entnommen und durch neue ersetzt.

Ein weiteres Beispiel für das austauschbare Modul 40 ist in Fig.4 schematisch dargestellt. Es besteht aus drei Teilen, nämlich zwei Kalotten 41,42 und einem linsenförmigen Gelenkteil 43, das schwimmend zwischen den Kalotten 41,42 liegt. Dieses austauschbare Modul 40 wird als lose Baueinheit zwischen Wirbelendplatten, wie beispielweise in Fig.3 gezeigt, zwischengelegt und mittels Schrauben 44 schräg an die jeweilige Platte angeschraubt.

Im Beispiel gemäß Fig.4 ist auch die Variante möglich, bei der das Gelenkteil 43 direkt zwischen zwei mit Kalotten ausgebildeten Wirbelendplatten eingelegt wird. Dieses ist ein Beispiel für ein einteiliges, gelenkiges austauschbares Modul. In diesem Fall wären die beiden Kalotten 41,42 als Wirbelendplatten ausgebildet und würden mittels der Schrauben 44 an den jeweiligen Wirbelkörper angeschraubt werden.

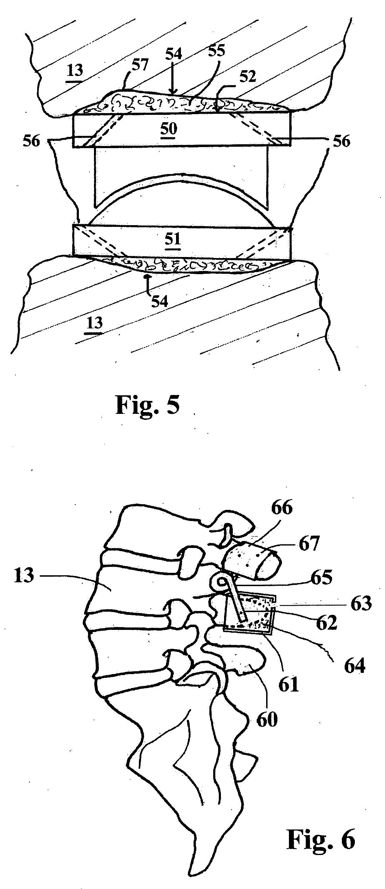

Die künstlichen Wirbelendplatten 50,51 gemäß Fig. 5 haben in der Regel wirbelkörperseitige ebene Anbindungsflächen 52 die nicht kongruent mit der angrenzenden, eher konkaven Oberfläche 54 des Wirbelkörpers 13 sind. Es verbleiben Hohlräume 57. Dadurch ergeben sich über die Flächen 52,54 unterschiedliche Druckverhältnisse, die rasch zu einer Lockerung der künstlichen Wirbelendplatten 50,51 bzw. des Implantats führen können. Um dieses zu verhindern sind in den Wirbelendplatten 50,51 Bohrungen oder Kanäle 56 vorgesehen, durch die nach dem Fixieren der Wirbelendplatten 50,51 an den jeweiligen Wirbelkörper 13 ein aushärtbares Material 55 in die Zwischenräume 57 eingespritzt wird.

Fig.6 zeigt beispielsweise eine Halterung für interspinöse Prothesen. Dargestellt ist eine Wirbelsäule im Lendenbereich von der Seite aus gesehen. Das Funktionsmodul ist als Schraubenfeder 65 dargestellt, dessen freien Enden 62 mit angrenzenden Dornfortsätzen 60 verbunden werden. Als Verbindungsplattform sind hier Hülsen 61,66 vorgesehen, die auf Dauer mit dem jeweiligen Dornfortsatz 60 verbunden sind. Die Hülse 66 ist beidseitig offen, sie wird über den Dornfortsatz gestülpt und mittels nicht näher dargestellten Krampen, Schrauben oder dergleichen am Dornfortsatz fixiert. Zwischenräume zwischen der Hülse 66 und der unebenen Dornfortsatzoberfläche werden mit einem seitlich eingespritzten aushärtbaren Material ausgefüllt. Die Hülse ist mit Mitteln, wie Schraubbohrungen 67, für die Verbindung von Funktionsmodulen 65 vorgesehen.

Die als dauerhafte Halterung dienende Hülse kann auch einseitig geschlossen sein, wie die im Längsschnitt dargestellte Hülse 61 zeigt. Hier ist für das Einspritzen des aushärtbaren Material 64 eine Öffnung 63 vorgesehen. Hülsen können auch für Querfortsätze von Wirbelkörpern vorgesehen werden.

Als aushärtbares Material kann jeder körperverträglicher Kunststoff Verwendung finden. Es eignen sich auch Material mit osteoconductiven oder osteoinductiven Eigenschaften.

Auch hülsenartige Halterungsmodule 61,66 sind vorzugsweise genormt.

## Patentansprüche

1. Modulare Bandscheibenprothese bestehend aus mindestens einer an einem Wirbelkörper implantierbarer Appositionsplatte und einem mit der bzw. den Appositionsplatte/n lösbar befestigbaren Zwischenmodul, wobei die Appositionsplatte/n mit Mitteln wie Stiften, Krampen, Zahnungen, ausgebildet ist bzw. sind derart, daß sie auf Dauer, d.h. Revisionen überstehend, am Wirbelkörper fixierbar ist bzw. sind, **dadurch gekennzeichnet, dass** die Appositionsplatte/n (10;33,34;50,51) mit Bohrungen (56) versehen ist/sind, durch die ein aushärtbares Material (55) in einen zwischen Platte und unebener Knochenoberfläche (54) verbleibenden Hohlraum (57) gespritzt werden kann.

2. Bandscheibenprothese nach Anspruch 1, **dadurch gekennzeichnet, daß** die Appositionsplatte/n (10;33,34;50,51) mit Mitteln (14;36;52,53;54) ausgestattet ist/sind, die eine sichere und lösbare Verbindung mit dem Zwischenmodul (20;40;56,58) gewährleisten und einen Postoperativen Austausch von Zwischenmodulen erlaubt.

3. Bandscheibenprothese nach einem der vorhergehenden Ansprüche, **dadurch gekennzeichnet, daß** die Appositionsplatte/n (10;33,34;50,51) mit Schraubbohrungen (35';36;61) für die lösbare Fixierung des Zwischenmoduls (20;40;56,58) und/oder für Hilfsmittel, wie Dämpfer, Zuggurtungsbänder (15), etc. versehen sind.

4. Bandscheibenprothese nach einem der vorhergehenden Ansprüche, **dadurch gekennzeichnet, dass** die Appositionsplatten (50,51) derart ausgebildet sind, dass sie bei einer Revision zur Versteifung der Eingriffstelle, nach Entfernung des Zwischenmoduls (56,58) direkt miteinander verbindbar sind.

## Claims

1. Modular intervertebral disc prosthesis consisting of at least one apposition plate which can be implanted on a vertebral body and of an intermediate module which can be detachably fastened to the apposition plate or plates, the apposition plate or plates being designed so as to comprise means such as pins, staples or toothings such that they can be fixed to the vertebral body permanently, i.e. so as to withstand repair operations, **characterised in that** the apposition plate or plates (10; 33, 34; 50, 51) is/are provided with holes (56), through which a curable material (55) can be injected into a hollow space (57) remaining between the plate and uneven bone surface (54).

2. Intervertebral disc prosthesis according to claim 1, **characterised in that** the apposition plate or plates (10; 33, 34; 50, 51) is/are equipped with means (14; 36; 52, 53; 54) which ensure a secure and detachable connection to the intermediate module (20; 40; 56, 58) and enable postoperative replacement of intermediate modules.

3. Intervertebral disc prosthesis according to any of the preceding claims, **characterised in that** the apposition plate or plates (10; 33, 34; 50, 51) are provided with threaded holes (35'; 36; 61) for detachably fixing the intermediate module (20; 40; 56, 58) and/or for aids such as dampers, tension cord bands (15), etc.

4. Intervertebral disc prosthesis according to any of the preceding claims, **characterised in that** the apposition plates (50, 51) are designed such that they can be directly interconnected in the event of a repair operation for reinforcing the point of intervention after removing the intermediate module (56, 58).

## Revendications

1. Prothèse de disque intervertébral modulaire constituée d'au moins une plaque d'apposition pouvant être implantée au niveau d'un corps vertébral et d'un module intermédiaire pouvant être fixé de manière amovible à la ou aux plaques d'apposition, la/les plaques d'apposition étant réalisées avec des moyens tels que des tiges, des crampons, des dentures, de telle manière qu'elle ou elles peut/peuvent être fixée(s) au niveau du corps vertébral à long terme, c'est-à-dire de manière à supporter des révisions, **caractérisée en ce que** la/les plaque(s) d'apposition (10 ; 33, 34 ; 50, 51) est/sont pourvue(s) d'alésages (56), à travers lesquels un matériau (55) durcissable peut être injecté dans une cavité (57) restant entre la plaque et une surface de l'os (54) non plane.

2. Prothèse de disque intervertébral selon la revendication 1, **caractérisée en ce que** la/les plaque(s) d'apposition (10 ; 33, 34 ; 50, 51) est/sont équipée(s) de moyens (14 ; 36 ; 52, 53 ; 54), qui garantissent une liaison fiable et amovible au module intermédiaire (20; 40 ; 56, 58) et qui permettent un remplacement postopératoire de modules intermédiaires.

3. Prothèse de disque intervertébral selon l'une quelconque des revendications précédentes, **caractérisée en ce que** la/les plaque(s) d'apposition (10 ; 33, 34 ; 50, 51) est/sont pourvue(s) d'alésages taraudés (35' ; 36; 61) pour la fixation amovible du module intermédiaire (20 ; 40 ; 56, 58) et/ou pour des moyens auxiliaires, tels que dés amortisseurs, des bandes de membrures de tension (15), etc.

4. Prothèse de disque intervertébral selon l'une quelconque des revendications précédentes, **caractérisée en ce que** les plaques d'apposition (50, 51) sont réalisées de telle manière qu'elles peuvent être reliées directement les unes aux autres lors d'une révision visant à renforcer l'emplacement de prise, après le retrait du module intermédiaire (56, 58).
